# EUROPEAN PATENT SPECIFICATION

(11) **EP 1 309 867 B1**
(45) Date of publication and mention of the grant of the patent: **11.10.2006**
(21) Application number: 01971884.0
(22) Date of filing: 07.08.2001
(51) Int. Cl.: G01N 33/58, G01N 33/558, G01N 33/543

(54) **CAPSULES ENCAPSULATING SOLID PARTICLES OF SIGNAL-GENERATING ORGANIC SUBSTANCES**
KAPSELN, ENTHALTEND FESTSTOFFPARTIKELN VON SIGNALERZEUGENDEN ORGANISCHEN SUBSTANZEN
CAPSULES RENFERMANT DES PARTICULES SOLIDES DE SUBSTANCES ORGANIQUES FOURNISSANT UN SIGNAL

(30) Priority: 08.08.2000 DE 10042023
(43) Date of publication of application: 14.05.2003
(73) Proprietor: 8Sens.Biognostic AG, 13125 Berlin (DE)
(72) Inventor: TRAU, Dieter, 16352 Basdorf (DE); RENNEBERG, Reinhard, Hong Kong Univ. Sci. & Techn., Kowloon (CN); CARUSO, Frank, 14476 Golm (DE); LEHMANN, Matthias, 13125 Berlin (DE)
(74) Representative: Ziebig, Marlene
(86) International application number: PCT/EP2001/009114
(87) International publication number: WO 2002/012888

(56) References cited:
- EP-A- 0 577 092
- WO-A-99/47252
- US-A- 5 593 843

## Description

The present invention refers to capsules encapsulating solid particles of signal-generating organic substances and carrying on the outer surface affinity molecules for specific recognition of and binding to target molecules in a sample. The invention is directed to the use of these capsules for signal production in the optical, electrochemical or chemical detection of target molecules. To obtain a signal the signal-generating organic substances are released and dissolved. A detection method and a kit are provided.

Bioassays like enzyme-linked immunoassays (ELISA), radioimmunoassays (RIA), fluorescence immunoassays (FIA) or immuno agglutination assays are well known in the art and play an important role in the detection of analytes in medical diagnosis, environmental analysis and food analysis. Bioassays are based on the interaction of a labelled biomolecule with an analyte to be detected. The label acts as an instrument to visualize the interaction. Different kinds of labels are known: enzymes in ELISAs, radio isotopes in RIAs, fluorophores in FIAs, liposomes, latex particles in immuno agglutination assays as well as dyes, mediators, gold particles and DNA.

The most important requirements for bioassays are specificity and sensitivity. The specificity is determined by the biorecognition molecule, e.g. the matching of the binding site of an antibody to its antigen (analyte) or the hybridisation of two complementary nucleic acid strands. The sensitivity of an bioassay is also influenced by the biorecognition molecule due to its affinity constant of its biointeraction. The label acts as a marker for the biomolecule and can be measured with different techniques: i) optically by the measurement of the absorption of a dye or the fluorescent light emitted by fluorophores or turbidimetric by the light scattering of agglutinated latex particles, ii) radioactively by the measurement of radio isotopes, iii) electrochemically by the measurement of mediators or electroactive substances.

The sensitivity of the bioassay is strongly determined by the nature of the label and the detection technique employed to measure the label concentration. The RIA technology, which is using radio isotopes as labels, is still the most sensitive method. This very powerful technique was introduced in 1959 by Yalow and Berson and represented a new era in analytical chemistry, diagnostics and medicine. Nevertheless this technique has the drawback that the risk of dangerous contaminations of people and environment can not be reduced to zero due to the radioactive isotopes used.

Therefore, non-radioactive methods were developed and improved with the aim to reach a comparable sensitivity. The importance of the optical methods based on fluorescence, luminescence and absorption spectroscopy was strengthened over the time and is still growing.

The ELISA technology uses enzymes as markers to amplify the signal. After the bioassay is performed, the biointeraction of the analyte and the probe is amplified by the production of a high number of dye molecules by one enzyme marker molecule. Enzymes like glucose oxidase (GOD, EC 1.1.3.4.), alkaline phosphatase (AP, EC 3.1.3.1) or peroxidase (POD, EC 1.11.1.7) with turnover numbers of 2000 substrate molecules per second (s⁻¹), 5.000 s⁻¹ and 10.000 s⁻¹ respectively, are used. Drawbacks of the ELISA technique are the high number of steps involved in the procedure and the additional time needed for substrate incubation.

Fluorescence methods are also employed in bioanalytics over years. They are of high interest. All fluorescence based techniques ensure a good sensitivity and a low detection limit of 10⁻⁸ to 10⁻¹⁸ M. Special techniques, e.g. "time resolved fluorescence", chemi- and bioluminescence or techniques based on the energy transfer between a dye and a fluorophore molecule can reach detection limits of 10⁻¹⁵ to 10⁻¹⁸ M.

The fluorescence-immunoassays known in the prior art use low molecular fluorescent labels with reactive functional linker groups (SOUTHWICK, P.L., *et al., Cytometry,* 11, pp.418-430, 1990, MUJUMDAR, R.B., *et al., Bioconjugate Chemistry,* 4, pp.105-111, 1993, MUJUMDER, R.B., *et al., Cytometry,* 10, pp.11-19, 1989), fluorescent and dye coloured particles (US 4.837.168; US 6.013.531; WO 95/08772) or fluorophore spiked dendrimeres (DE 197 03 718).

It is also known from the prior art to employ marker-loaded liposomes for signal amplification in immunoassays (US 5.756.362, 4.874.710, 4.703.017). The sensivity of these methods is limited by the limitation of the amount of marker substances which may be incorporated into the liposomes only in solubilized form. A further drawback of using labelled liposomes is the limited stability of liposomes.

It was the object of the present invention to provide a detection method for target molecules in a sample which ensures a very good sensitivity, a low detection limit and an enhancement of the detection signal related to the amount of the target molecule in comparison with the methods known in the prior art. Preferably, the method of the invention should be suited for optical detection, especially in fluorescence immunoassays.

It was a further object of the invention to provide a kit for optical, electrochemical or chemical detection of target molecules.

It was also an object of the invention to provide labelled biomolecules which are easy to prepare and applicable in the bioassays of the invention, preferably in fluorescence immunoassays.

The problem underlying the invention is solved by a method for optical, electrochemical or chemical detection of one or more target molecules in a sample using affinity-based interactions between the target molecule and an affinity molecule for specific recognition of the target molecule comprising the steps
i) incubating the target molecules with capsules encapsulating solid particles of organic signal-generating substances and carrying on their outer surface the corresponding affinity molecules,
ii) separating the resulting target-affinity molecule complex from the capsules with unreacted affinity molecules on their surface,
iii) releasing and dissolving the encapsulated solid particles of the signal-generating substances by treating the obtained target-affinity complex with physical or chemical means,
iv) detecting or quantifying the signal which is generated by the released and dissolved signal-generating substances and which is directly or indirectly related to the amount of the target molecules.

Due to the capsules of the invention which encapsulate solid organic signal-generating substances in high quantities and which are released and dissolved during the detection process a high signal amplification is achieved.

The capsules according to claims 1 to 19 are also an object of the invention.

According to the invention the capsules carry affinity molecules on the outer surface which specifically recognize and bind to the target molecules. By "target molecule" is meant the analyte to be detected or the analyte linked to a molecule which is capable to bind to the affinity molecules on the capsule surface.

Depending on the target molecules to be detected the affinity molecules are selected from the group of peptides or proteins, nucleic acids, carbohydrates, ligands with low molecular weight and molecular imprinted polymers (MIPs) or mixtures thereof. As peptides or proteins antibodies, receptors, antigens, lectins, avidins, oligopeptides, lipopoteins, glycoproteins, peptide hormones and allergenes or parts thereof may be used. The nucleic acids which may also be affinity molecules on the capsule surface are selected from the group of single or double stranded DNA, RNAs, oligonucleotides, ribozymes, aptamers and parts thereof. Also carbohydrates which are selected from the group of mono, oligo and polysaccharides, glycolipids, proteo-polysaccharides and parts of thereof may be the affinity molecules. In accordance with the invention low molecular weight ligands are biotin or biotin derivatives, a steroid or hormone, a cofactor or coenzyme, activator, inhibitor, pseudosubstrate or prosthetic group of an enzyme, a drug, a pesticide, an allergen or digoxine or a haptene.

The affinity molecules are conjugated to the outer surface of the capsules, e.g. by van der Waals forces, hydrogen bonds or electrostatic interactions, or they are directly or via linker molecules covalently bound to the outer surface of the capsules, where the linker molecule is usually a biomolecule, preferably avidin, streptavidin, neutravidin, protein A, protein G, lectin or a low molecular crosslinker.

The capsules encapsulating solid signal-generating organic substances which are used in the detection method of the invention are prepared by treating low water soluble or water insoluble uncharged solid signal-generating organic substances with an aqueous solution of an ionic detergent whereby the amphiphilic substance is arranged on the surface of the solid signal-generating substances rendering them susceptible to the subsequent coating with a layer of a charged polyelectrolyte or with a multilayer comprising alternating layers of oppositely charged polyelectrolytes or rendering them susceptible to the subsequent coating with a single layer of a substance bearing functional groups for covalent coupling of a second layer or multiple layers.
The solid particles in the suitable size are generated by different well known methods, e.g. milling or spray drying.

In the first step of the process the uncharged solid signal-producing organic particles are treated with an amphiphilic substance, which imparts an electrical charge on the surface of the particle material. The treatment of the solid signal generating particles with the surfactant is also possible during a wet milling process.

In the second step the particles coated with amphiphilic substance are coated with a polyelectrolyte which is oppositely charged with respect to the surface of the particle materials. For the formation of multilayers the signal-producing particles are subsequently treated with oppositely charged polyelectrolytes, i.e. alternatingly with cationic and anionic polyelectrolytes. Polymer layers self-assemble onto the pre-charged solid templates by means of electrostatic layer-by-layer deposition, thus forming a multilayered polymeric shell around the solid cores.
The second step can also be performed by a single layer of substances bearing functional groups for covalent coupling of a second or more layers not using electrostatic deposition.

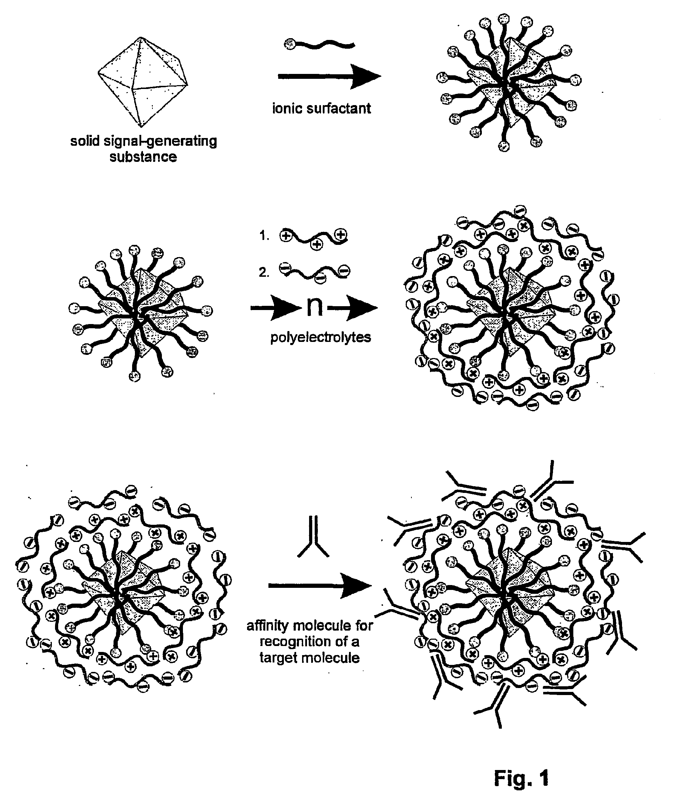

In a last step the surface of the obtained capsules is modified with affinity molecules in accordance with the target molecule which should be detected (compare Fig. 1). According to the invention the capsules are incubated with the affinity molecules, e. g. immunoglobulines. In an other embodiment of the invention the affinity molecules are covalently bound to the capsules by chemical reactions, e. g. after EDC/NHS activation of the particles with an outermost layer of the poly-electrolyte polyacrylic acid or alginic acid.

It is also possible to react the affinity molecules with linker molecules which have at least two functional groups and are able to bind to the capsule and to the affinity molecule, e. g. by using a homo- or hetero bifunctional crosslinker like DSS, DTSSP or SMCC, MBS, respectively.

The described encapsulation process allows to include a wide range and high amounts of signal-generating organic substances (10⁷ to 10⁹ molecules per particle, depending on the size and density of the particle and the molecular weight of the substance). Depending on the desired detection method low molecular substances selected from fluorophores, luminophores, chromophores, enzyme substrates, prosthetic groups, or redox active substances selected from redox mediators, electrodeactive substances, metal salts may be encapsulated. Also high molecular substances selected from the group of enzymes and their precursors, bioluminogenic and fluorogenic proteins, nucleic acids, ribozymes and aptamers may be the signal-generating substances which are encapsulated.

According to the invention the encapsulated solid particles of signal-generating substances are crystals, amorphous or lyophilised particles, spray dried particles, milled particles or mixtures thereof. In a preferred embodiment of the invention the encapsulated solid particles have a particle size from 10 nm to 10 µm, preferably smaller than 1 µm.

To prepare the capsules of the invention as amphiphilic substance any substance can be used which has ionic hydrophilic and hydrophobic groups. Preferably, ionic surfactants, phospholipids and/or amphiphilic polyelectrolytes are used. Amphiphilic polyelectrolytes, for example, are polyelectrolytes comprising a charged group as hydrophilic group and a hydrophobic group, e.g. aromatic groups. It is preferred to use a cationic or/and anionic surfactant. Examples of suitable cationic surfactants are quaternary ammonium salts (R₄N⁺X⁻), especially didodecyldimethylammonium bromide (DDDAB), alkyltrimethylammonium bromides, especially dodecyltrimethylammonium bromide or palmityl trimethylammonium bromide or N-alkylpyridinium salts or tertiary amines (R₃NH⁺X⁻), especially cholesteryl-3β-N-(dimethylaminoethyl)-carbamate or mixtures thereof, wherein X⁻ means a counteranion, e.g. a halide. Examples of suitable anionic surfactants are alkyl sulfonate (R-SO₃M), especially dodecyl sulfate, e.g. sodium dodecyl sulfate (SDS), lauryl sulfate or olefin sulfonate (R-SO₃M), especially sodium-n-dodecyl-benzene sulfonate or alkyl sulfates (R-OSO₃M) or fatty acids (R-COOM), especially dodecanoic acid sodium salt or phosphoric acid or cholic acids or fluoroorganics, especially lithium-3-[2-(perfluoroalkyl)ethylthio] propionate or mixtures thereof. Particularly preferred are surfactants having 6 to 30 carbons in their alkyl or olefin groups.

Further, it is preferred to use a polymeric substance which provides charged groups and hydrophobic sites as amphiphilic substance. In a preferred embodiment poly(styrene sulfonate) (PSS) is used.

Polyelectrolytes, generally, are understood as polymers having ionically dissociable groups, which can be a component or substituent of the polymer chain. Usually, the number of these ionically dissociable groups in polyelectrolytes is so large that the polymers in dissociated form (also called polyions) are water-soluble. The term polyelectrolytes is understood in this context to cover also ionomers, wherein the concentration of ionic groups is not sufficient for water-solubility, however, which have sufficient charges for undergoing self-assembly. However, the shell preferably comprises "true" polyelectrolytes, i.e. water-soluble polyelectrolytes. Depending on the kind of dissociable groups polyelectrolytes are classified as polyacids and polybases.

Dissociated polyacids form polyanions, with protons being split off, which can be inorganic, organic and biopolymers. Examples of polyacids are polyphosphoric acid, polyvinylsulfuric acid, polyvinylsulfonic acid, polyvinylphosphonic acid and polyacrylic acid. Examples of the correspoding salts which are also called polysalts, are polyphosphate, polysulfate, polysulfonate, polyphosphonate and polyacrylate.

Polybases contain groups which are capable of accepting protons, e.g. by reaction with acids, with a salt being formed. Examples of polybases having dissociable groups within their backbone and/or side groups are polyallylamine, polyethylimine, polyvinylamine and polyvinylpyridine. By accepting protons polybases form polycations.

Suitable according to the invention are also biopolymers such as alginic acid, gummi arabicum, nucleic acids, pectins, peptides, proteins and others as well as chemically modified biopolymers such as carboxymethyl cellulose and lignin sulfonates as well as synthetic polymers such as polymethacrylic acid, polyvinylsulfonic acid, polyvinylphosphonic acid and polyethylenimine.

Linear or branched polyelectrolytes can be used. Using branched polyelectrolytes leads to less compact polyelectrolyte multilayers having a higher degree of wall porosity. To increase capsule stability polyelectrolyte molecules can be crosslinked within or/and between the individual layers, e.g. by crosslinking amino groups with aldehydes.

Basically, there are no limitations with regard to the polyelectrolytes and ionomers, respectively, to be used, as long as the molecules bear sufficiently high charge or/and are capable of binding with the layer beneath via other kinds of interactions, e.g. hydrogen bonds and/or hydrophobic interactions.

Suitable polyelectrolytes, thus, are both low-molecular polyelectrolytes and polyions, respectively, e.g. having molecular weights of a few hundred Daltons, up to macromolecular polyelectrolytes, e.g. polyelectrolytes of biological origin, having a molecular weight of several thousand to million Daltons.

Further examples of an organic polymer as bioelectrolyte are biodegradable polymers such as polyglycolic acid (PGA), polylactic acid (PLA), polyamides, poly-2-hydroxy-butyrate (PHB), polycaprolactone (PCL), poly(lactic-co-glycolic)acid (PLGA), fluorescent-labelled polymers, conducting polymers, liquid crystal polymers, photoconducting polymers, photochromic polymers and their copolymers and/or mixtures thereof. Examples of biopolymers preferred as polyelectrolyte are polyamino acids, in particular peptides, S-layer proteins, polycarbohydrates such as dextrin, pectin, alginate, glycogen, amylose, chitin, chondrotin, hyaluronic acid, polynucleotides, such as DNA, RNA, oligonucleotides or/and modified biopolymers such carboxymethyl cellulose, carboxymethyl dextran or lignin sulfonates. Preferred examples of inorganic polymers as polyelectrolyte are polysilanes, polysilanoles, polyphosphazenes, polysulfazenes, polysulfides and/or polyphosphates.

It is also possible to deposit charged nanoparticles or biomolecules as capsule material.

The preparation of the capsules of the invention is preferably carried out so that excess material of the starting substances used in the individual steps is separated after each treatment step.
For example, an aqueous dispersion of the template particles is formed first, to which an aqueous solution of the amphiphilic substance is added. After separating any excess amphiphilic molecules a first polyelectrolyte species is then added to build up the first polyelectrolyte shell. After separating any excess polyelectrolyte molecules the oppositely charged polyelectrolyte species used for building up the next layer is then added. Subsequently, oppositely charged layers of polyelectrolyte molecules are applied in turn, where for each layer having the same charge identical or different polyelectrolyte species or mixtures of polyelectrolyte species can be selected and between each incubation step a purification step is carried out. Strategies for coating the capsules with affinity molecules are described in example 4. In accordance with the invention the binding of biotinylated molecules to avidin precoated capsules is preferably employed.

In a further embodiment of the invention a single layer of substances (e.g. proteins, peptides, nucleotides, DNA, RNA) bearing functional groups (e.g. -COOH, -NH₂, -SH) is coated onto the solid organic particles having the amphiphilic substance on their surface for covalent coupling of a second or multiple substances being polyelectrolytes or other substances (proteins, peptides, nucleotides etc.) bearing functional groups like for instance -COOH, -NH₂, -SH and so forming a second layer or more layers being covalently linked. According to this embodiment of the invention the substances bearing functional groups are organic polymers, biopolymers or mixtures thereof, preferably substances bearing -COOR, -NRR¹, -SR, -OR, -SSR, -C(O)R, -OC(OH)RR¹ or -SC(O)R groups, wherein R and R¹ are independently from one another hydrogen or a linear or branched alkyl group. These substances may also be polyelectrolytes bearing said functional groups and thus allowing adsorption and/or covalent linking of suited substances to form a or more further layers.

According to the invention the capsules prepared as described above are excellently applicable in in-vitro bioassays for optical, electrochemical or chemical detection of target molecules. In a first step of the detection method of the invention the target molecules are incubated with the capsules of the invention carrying on the outer surface affinity molecules which specifically recognize the target molecules for a time sufficient to result in a target-affinity molecule complex. In a preferred embodiment of the invention the target molecules are bound directly or via another affinity molecule to a solid phase, e.g. a microtiter plate or a nitrocellulose pad.

In the second step of the detection procedure the resulting target-affinity molecule-capsule complex is separated from the unreacted affinity molecules, that means from the capsules with unreacted affinity molecules on their surface.

In the next process step the encapsulated solid particles of the signal-generating organic substances are released from the capsules into medium using physical or chemical means. In an embodiment of the invention the release is achieved by adding reagents which are suitable for dissolving the uncharged solid core substances, e.g. an organic solvent in which the material is soluble or an acid or alkaline solvent . According to the invention, dissolution of the template particles can be effected in a gentle manner during a short incubation period, e.g. 1 min to 1 h at room temperature. The templates disintegrate almost completely, as no residue of the particles can be detected any longer even when inspecting the remaining shells by an electron microscope. In a preferred embodiment the capsules are treated with an organic solvent or with an enzyme or ribozyme or by changing the pH value or ionic strength value. Preferred organic solvents which may be employed are selected from the group of alcohols, especially ethanol and methanol, ketones, especially acetone, esters, especially ethylester, aromates, especially toluene, sulfoxides, especially DMSO and ethers, especially dimethylether, chloroform or from mixtures of organic solvents with one another or with water.

The disintegration of the encapsulated solid signal-generating substances may also be achieved by physical means, preferably by ultrasonic disintegration, electric impulse or osmotic shock.

The last step of the detection method of the invention encompasses the detection or the measurement of the signal which is generated by the released and dissolved signal-generating organic substances and which is directly or indirectly related to the amount of the target molecules.

In one embodiment of the invention the detection of one or more target molecules in a sample may be carried out in a fluorescence immunoassay. In this case, the encapsulated signal-generating substances are fluorophores, e.g. cyanine, carbocyanine, rhodamine, xanthene and diazo-dye based fluorescent substances as well as small fluorescent aromatic and heteroaromatic molecules such as perylene, pyrene, phenanthrene and oxazol. A condensed description of more than 500 dyes, including fluorophores, is given in: The Sigma-Aldrich Handbook of Stains, Dyes and Indicators; Floyd J.Green; Aldrich Chemical Company Inc., 1991.

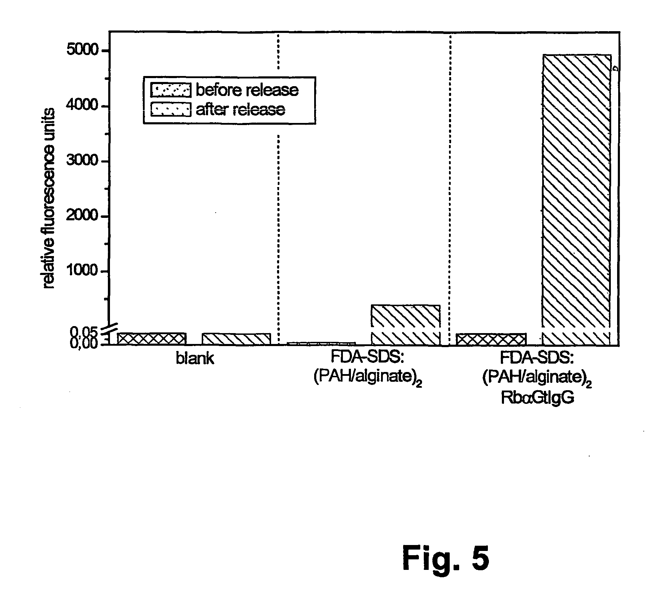

The fluorescence immunoassay is a preferred embodiment of the present invention. Due to the possible encapsulation of high amounts of solid fluorophore particles a high fluorophore/affinity molecule ratio is achieved. Due to the dissolution in a volume of solvent a high quantum yield is obtained. No quenching effects occur. As it is seen for instance from Example 3 and Fig. 5 of the present description a remarkable fluorescence signal amplification is detectable after releasing and dissolving the fluorophore particles from the capsules of the invention.

In a further embodiment of the invention the detection of one or more target molecules in a sample may also be carried out with a visible dye encapsulated in the capsules of the invention, preferably cyanine, pyrazolone, anthraquinone, anthroloine, carbocyanine, rhodamine, xanthene, carotenoid and diazo- and monoazo, oxazine, indigoid, riboflavine based dye substances as well as small fluorescent aromatic and heteroaromatic molecules such as perylene, pyrene, phenanthrene and oxazol.

In a further embodiment of the invention the detection may be carried out similar to an ELISA and an enzyme, e.g. horse-radish peroxidase or glucose oxidase, is the encapsulated signal generating substance. The corresponding substrate may be contained in the rupturing buffer, used for the destruction of the capsules and release of the enzyme.

According to the invention immunoagglutination of capsules is a suitable way to precipitate capsules bound to an analyte and separate them from unreacted capsules, that means capsules not bound to a target molecule or analyte.

The present invention further provides a method for detecting or quantifying an analyte (or analytes) using an electrochemical detection of an electroactive substance, an electro-inactive enzyme substrate or an enzyme as signal-generating substances.

The electrochemical measurement is performed with a sensor system comprising a working, reference and counter electrode and an electrolyte solution. This can be micro or macro thick-film, thin-film electrodes or conventional rod electrodes. In the amperometric approach, a potential is established allowing the reduction or oxidation of the electroactive substance. In the potentiometric approach, the electrochemical device described above is used unless it has an indicator and reference electrode combined and measures the potential shift caused by an electroactive substance (e.g. ions).

In amperometry, well-known mediators might be used as electroactive substances being oxidized or reduced at low potential. Depending on the nature of the analyte, the working electrode carries antibodies or antigens immobilized by different methods to the electrode surface or to a membrane covering the surface of the working electrode.

In the case of detecting a high-molecular antigen (for example, the early infarction marker Fatty Acid-Binding Protein, FABP), catcher antibodies against the antigen (FABP) are immobilized at the working electrode. A blood, plasma or serum sample (containing the antigen FABP) is added to the electrode together with capsules containing solid electroactive substances encapsulated and detector antibodies against antigen (FABP) at the capsule surface. The substances form a "sandwich" at the working electrode: catcher antibody - antigen(FABP) - detector antibody - encapsulated electroactive substance. After this, the electrode system is washed with neutral buffer to wash away unbound substances and sample constituents and organic solvents (e. g. DMSO or ethanol) are added to facilitate the release and dissolution of the electroactive substance near the electrode surface. A huge oxidation current is generated being proportional to the amount of electroactive substance released. The sensor system can be calibrated before use by different amounts of antigen and equal amounts of encapsulated electroactive substances.

The sensitivity of the assay can be even more increased if an enzyme in solid state is being encapsulated (for example, alkaline phosphatase, AP) for signal generation. An electrode-inactive substrate (like p-amino phenylphosphate, pAPP) is added in high concentration together with the rupturing reagent to release the enzyme. The product (p-aminophenol) is formed enzymatically, being oxidized subsequently at the working electrode.

For low-molecular antigens (haptens) which do not allow formation of sandwich configurations, the described systems can be used, if a competition principle is applied. In this case, the capsules have to be labelled by the antigen (or antigen analogue) and have to compete for the antibody bound to the surface of the working electrode. It can also be performed by binding the antigen (or antigen analogue) to the electrode and using antibody-labelled capsules which will then compete for the free analyte (antigen) (to be detected) and the antigen bound to the surface. In both cases, the electrode signal will be indirectly proportional to the analyte concentration (similar to ELISA and RIA).

In a further embodiment of the invention the capsules carry a chemical substance which can act as a catalyst (e.g. dibenzoylperoxide for the initiation of a polymerization reaction of a monomer, e.g. acrylic acid) or an educt for a chemical reaction (e.g. a coupling reagent for a diazonium salt formation being suitable to react in a diazonium reaction to form a dye or fluorescent product).

Further, the invention provides an immunochromatographic method (lateral flow test) for detecting or quantifying an analyte. As an example a higher-molecular antigen like FABP is selected which has to be detected in a plasma sample.

The sample is added to one end of an absorbent material of the testing device. The fluid is migrating to the other end by capillary forces enhanced by a sucking pad positioned at the other end. Detector antibodies labelled with capsules (containing the solid signal-generating substance) are loosely bound in excess at the starting point of the testing device.

The signal-generating substance can be a fluorescent dye, a visible dye, a bioluminescent or chemiluminescent material, or an enzyme.

The antigen (FABP) from the sample is interacting with the detector antibodies and migrating to the other end of the device carrying along the capsules. A sandwich is formed with catcher antibodies in the indicator region (dot or stripe) near the sucking pad. Here, catcher antibodies are more or less tightly bound to the absorbent material and therefore cannot migrate. The more analyte is present in the sample, the more sandwich structures are formed and the more capsules are bound in the indicator region. The signal-generating substance can be released by dipping the whole device into solvent (e.g. ethanol) or by dropping on solvent to the indicator region or by incorporating a capsule-lysing solvent into the device. The signal generated almost immediately can be detected with naked eye or, for quantification, using an optical reading device. In case of signal-gerneration by enzymes the according substrates have to be added.

A control region is placed behind the indicator region. It shows whether the dipstick is properly working and contains antibodies (a dot or stripe) tightly bound to the absorbent material and directed against detector antibodies. In any case, a part of the detector antibodies (being labelled with capsules) is not bound to the indicator region and proceeds with migrating. The control signal will be released at the same time as in the indicator region. Instead of the anti-detector antibodies, also the tightly-bound antigen (or antigen analogues) may be used for creating the control signal.

The dipstick can be modified for low-molecular substances using the competition principle. Instead of lateral flow, also a vertical flow device can be constructed. If several antigens are to be measured, the appropriate antibodies have to be used and different signal-generating substances have to be used for the different analytes accordingly.

The invention also relates to a kit for optical, electrochemical or chemical detection of target molecules in a sample comprising capsules encapsulating solid particles of signal-generating organic substances and carrying on the outer surface affinity molecules for specific recognition and binding to a target molecule. These capsules also may be supplied in lyophilized form, from the user easy to reconstitute with water. Optionally, the kit may also contain a dipstick as described above.

In a further embodiment the kit of the invention comprises
a) capsules encapsulating solid particles of signal-generating organic substances being designated to bind affinity molecules
b) agents for the modification of affinity molecules to make them suitable to bind to the surface of the capsules
c) agents for performing the binding reaction between the capsules and the affinity molecules.

In a specially preferred variant of this embodiment the capsules a) may contain a dye and are chemically preactivated, e.g. by carrying N-succinimide activated esters on their surface for the covalent coupling to an affinity molecule, e.g. immunoglobulines. As agents b) the kit may contain e.g. biotinylation reagents (3-sulfo-N-hydroxy succinimide biotin) for the biotinylation of an affinity molecule, making it suitable to bind to avidin precoated capsules. As agents c) the kit may contain for instance homo- or bifunctional cross linkers. It is also possible, that the kit may contain a dipstick as described above.

The invention is further illustrated by the following examples and figures.
Figure 1 schematically shows the encapsulation of the signal-generating solid particles in polyelectrolyte layers. In step 1 the uncharged particles are coated by the self-assembly of charged surfactant molecules, rendering them water dispersible and hence amenable to subsequent coating with polyelectrolyte layers (step 2). Each polyelectrolyte layer deposited has an opposite charge to that already adsorbed. In step 3 an affinity molecule which specifically recognizes a target molecule is conjugated or bound to the outer surface of the polyelectrolyte capsule.

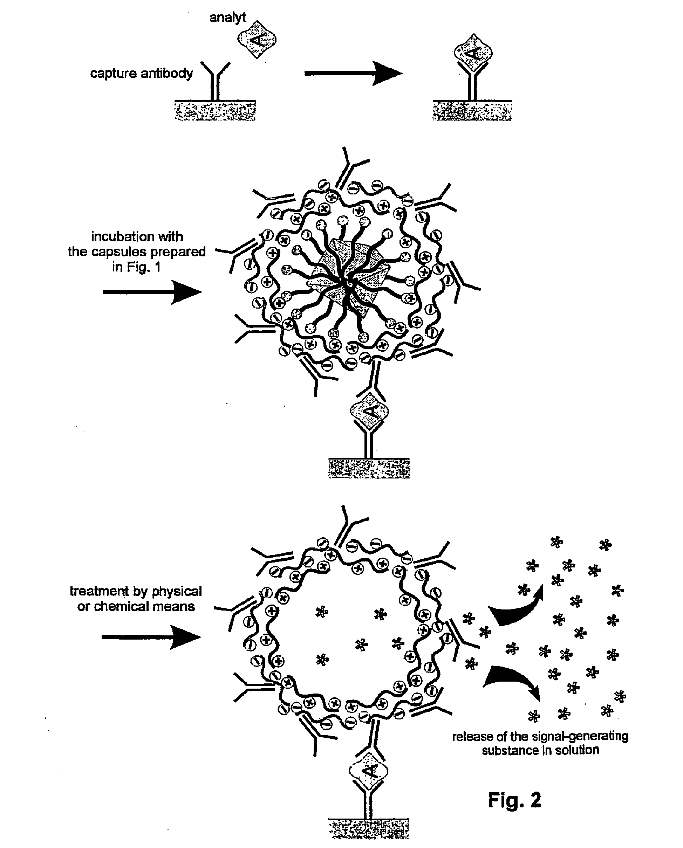

Figure 2 schematically shows one embodiment of the use of the capsules prepared according to the invention in a bioassay for detection of a target molecule (analyte). In a first step the analyte is immobilized via a capture antibody on a solid phase. In the second step the analyte is incubated with the capsules prepared according to the invention. The resulting complex is treated by physical or chemical means (step 3), so that the signal-generating particles are released into and dissolved in solution to be detected in the last step.

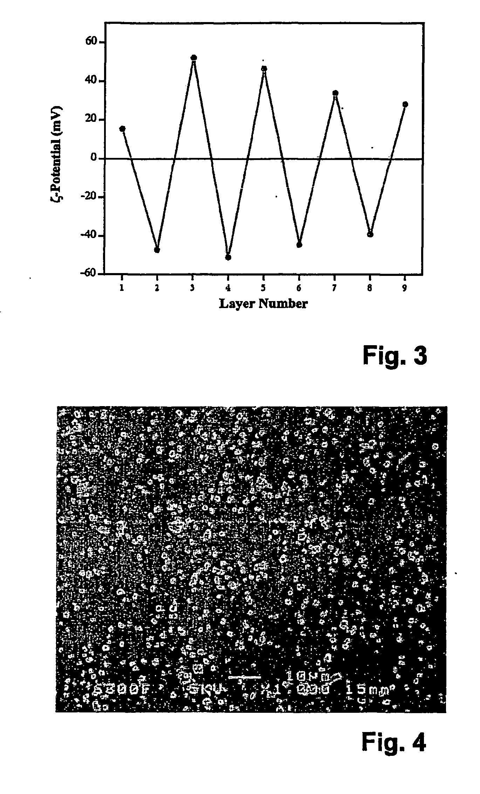

Figure 3 shows the electrophoretic mobility measurement of FDA/DPPC/(PSS/PAH)₄ capsules with ζ-Potentials as a function of polyelectrolyte layer number of FDA/DPPC/(PSS/PAH)₄ capsules. The FDA particles were precoated with a DPPC layer prior to the first PSS deposition. The even layer numbers correspond to PSS adsorption and the odd layer numbers (except for the first one) to PAH adsorption.
Figure 4 shows the SEM image of FDA/DPPC/(PSS/PAH)₄ capsules.
   The sizes of FDA particles milled and suspended in DPPC solution were in the range of 1~3 µm. After assembled with 4 bilayers of PSS/PAH, the particles show little aggregation.
Figure 5 shows the fluorescence immunoassay for detection of goat IgG using rabbit anti goat IgG conjugated to capsules built up by layers of FDA/SDS, /(PAH/alginate)₂ as label. Before the addition of DMSO/NaOH, the fluorescence signals were negligible. After the addition of DMSO/NaOH, the fluorescence signals compared to control were enhanced to more than 5,000 times.

### Examples

### Example 1

### Preparation of polyelectrolyte capsules encapsulating solid FDA particles as signal-generating substance

Particles of fluorescein diacetate (FDA) (from Sigma) were encapsulated as follows: 50 mg finely milled FDA was thoroughly mixed with 12 mL of 0.4 % DL-α-phosphatidylcholine dipalmitoyl (DPPC, from Sigma) or sodium dodecyl sulfate (SDS) solution. The suspension was allowed to stand for 15 min to settle down the big particles. The turbid white supernatant was extracted and concentrated to 2 mL by centrifugation and washed with water once. The resulting FDA particles then underwent the layer-by-layer assembly procedure with polyelectrolytes PSS and PAH, alternatively. The polycation, poly(allylamine hydrochloride) (PAH), *M*_{*w*} 15,000, and the polyanion, poly(sodium 4-styrenesulfonate) (PSS), *M*_{*w*} 70,000 were all purchased from Aldrich. PSS was dialyzed against Milli-Q water (*M*_{*w*} cut-off 14,000) and lyophilized before use. The water used in all experiments was prepared in a Millipore Milli-Q Plus 185 purification system and had a resistivity higher than 18.2 MΩ cm. All reagents used (except PSS) have been used as received from the producer.

Typically, 1 mL of PSS solution (containing 5 mg mL⁻¹ PSS and 0.5 M NaCl) was added to 0.5 mL DPPC pretreated FDA particle suspension. The suspension was shaken at constant intervals. After 15 min allowed for maximum adsorption, the suspension was centrifuged at 5,000 rpm for 3 min. Then the supernatant was removed and the precipitate washed with water to remove unadsorbed polyelectrolyte. Then 1 mL of PAH solution (containing 5 mg mL⁻¹ PAH and 0.5 M NaCl) was added to form a consecutive layer on the capsule surface. The centrifugation/washing steps and the consequent adsorption of oppositely charged polyelectrolytes were repeated. The capsules obtained were then referred to as FDA/DPPC/(PSS/PAH)ₙ, especially with n=4.

The electrophoretic mobility of coated FDA crystals was measured with a Malvern Zetasizer 4 by taking the average of 5 measurements at the stationary level. ζ-Potential was obtained from the relation *ζ=*uη/ε, where η and ε are the viscosity and permittivity of the testing solution, respectively (compare Fig. 3).

Scanning Electron Microscopy (SEM) measurements were performed with an instrument operated at an acceleration voltage of 5 kV. SEM samples (on glass cover slide) were sputter-coated with about 20 nm of Au (compare Fig. 4).

### Example 2

### FDA containing capsules used for immunoassay

Particles of fluorescein diacetate (FDA) from Sigma were encapsulated as follows:
1 ml sodium dodecyl sulfate (SDS) solution was added to 100 mg FDA. The suspension was mixed thoroughly and allowed to stand for 10 sec for sedimentation of large particles. After this, the supernatant was extracted and thoroughly mixed again. After 20 sec (sedimentation of the medium-sized particles) the supernatant was extracted a second time. This supernatant was centrifuged for 10 min at 9000 rpm (8000x g). Then, the resulting FDA particles were used for a layer-by-layer assembly procedure with the positivly charged polyelectrolyte PAH and the negativley charged biopolymer alginic acid, alternatively.

The polycation, poly(allylamine hydrochloride) (PAH), M_{w} 15.000, and the polyanion, alginate, approx. 250 cps, were all purchased from Aldrich. PAH and alginate were sterilized by filtration (0.22µm). The water used in all experiments had a resistivity higher than 18.2 MΩ x cm. All reagents have been used as received from the producer.

Typically, 1 mL of PAH (containing 5 mg mL⁻¹ PAH and 0.5 M NaCl) was added to 1ml SDS pretreated FDA particle suspension. The suspension was shaken for 15 min. After 15 min the suspension was centrifuged at 8000 g for 10 min. Afterwards the supernatant was removed and the precipitate washed with water for three times removing excessive unadsorbed polyelectrolyte. Then, 1 mL of alginate solution (containing 5 mg mL⁻¹ and 0.5 M NaCl) was added to form a consecutive layer on the capsule surface. The centrifugation/washing steps of oppositely charged polyelectrolytes were repeated. The capsules obtained were then referred as FDA/SDS/(PAH/Alginat)ₙ, especially with n ≥ 2.

### Antibody coupling to the outer surface of FDA/SDS/(PAH/Alginat)₂ capsules

The covalent coupling of rabbit anti goat immunoglobulin G (RbαGtIgG) onto the capsules was carried out as follows:
250 µL FDA/SDS/(PAH/Alg)₂ capsules were diluted in 250 µL 10 mM 2-[N-morpholino] ethansulfonic acid (MES) buffer pH 5.8 and activated with 40 mg 1-Ethyl-3-(3-dimethylaminopropyl)carbodiimide-HCl (EDC) (from Pierce). This solution was mixed for 15 min. In the meantime 200 µl RbαGtIgG, 2 mg mL⁻¹ (from Sigma-Aldrich) were diluted in 300 µL PBS pH 7.2 containing 0.05% BSA. This solution was mixed with the above described activated capsules and incubated for 2 h by constant shaking.

After this, the solution was centrifuged (700 x g for 5 min) and uncoupled antibodies were washed away with MES buffer for three times.

To detect the RbαGtIgG on the outer surface of the capsules made above a fluorescence immunoassay using goat immunoglobulin G (GtIgG from Sigma-Aldrich) as analyte was made.

A polystyrene 96-well microtiterplate (Nunc Maxi sorp) was coated overnight at 4°C with GtIgG (200ng/well) in natriumhydrogencarbonate/ binatriumcarbonate buffer pH 9.6. After washing (washing buffer containing PBS, 0.1%BSA) twice, the plate was blocked with 0.5% bovine serum albumin (BSA) (fraction V, obtained from Sigma-Aldrich) at 37°C for 2 h. In the next step the above washing procedure was repeated and followed by filling the plate with the capsules which were previously diluted 1:4. After incubating for 2 h at 37°C the plate was washed four times.

The first measurement of the whole plate was accomplished using a fluorescence microplate reader (Dynex) with washing buffer in each plate.This measurement was done having a controll signal. No significant signal was measured.

Afterwards the plate was washed twice again and than 100µL of DMSO/0.5 M NaOH solution (1:1) were added to each well. The plate was examined again by fluorescence microplate reader (Dynex). In comparison to the control signal a enhancement of more than 5000fold was obtained as shown in Figure 5.

### Example 3

### Covalent linking of the separate consecutive layers of the capsuls

Particles of fluorescein diacetate (FDA) from Sigma were encapsulated as follows (cf. Example 2):

1 mL sodium dodecyle sulfate (SDS) solution was added to 100 mg FDA. The suspension was mixed thoroughly and allowed to stand for 10 sec for sedimentation of large particles. After this, the supernatant was extracted and thoroughly mixed again. After 20 sec (sedimentation of the medium-sized particles) the supernatant was extracted a second time. This supernatant was centrifuged for 10 min at 9000 rpm (8000x g). Then, the resulting FDA particles were used for a layer-by-layer assembly procedure where two neighbouring layers were crosslinked.

The first layer applied on the above-mentioned particles was the protein hemoglobin (having an IP of 6.8; in MES buffer, pH 5.8, therefore being positively charged at this pH). After washing three times, the following layer was covalently linked to the first one, using 1-ethyl-3-(3-dimethylamino-propyl)carbodiimide (EDC).

In this example, alginate (5 mg/mL, 0.5 M NaCl) activated in 80 mg EDC was used as second layer. Now, the activated alginate solution was added to the above solution of capsules and thoroughly mixed and incubated for two hours. After this, the solution was washed three times with MES buffer (pH 5.8) and centrifuged for 10 min at 9000 rpm (8000x g).

The last step comprised the covalent coupling of antibodies (RbαGtIgG) to the above capsules via EDC (cf. Example 2).

### Example 4

### Alternative Conjugation strategies to bind biointer-active molecules, e.g. Immunglobulin G to the capsule surface:

### Direct adsorption of IgG

1 ml of the fluorophore particle suspension (5 % w/v) was incubated with 100 µl of a monoclonal anti-FABP Antibody (2 mg/ml), for 2 hours at room temperature and 12 hours at 4 °C. The excess of antibody was removed by repeated centrifugation and wash cycles, the label was finally resuspended in PBS.

### Coating of IgG via neutravidine; A method which is applicable to use the capsules in a kit

a) Biotinylation of antibody: 0.2 mg Biotin-X-NHS (Pierce, USA) in 100 µl coating buffer is added to a solution of 1 mg monoclonal anti-hCG-beta antibody (Clone ME 106, Dunn Labortechnik, Asbach, Germany) in 1 ml coating buffer. The mixture is incubated for 3 hours at room temperature. The not reacted biotin is separated from the antibody by repeated centrifugation steps in a centricon seperation unit (30 kDa, Centricon, USA). The buffer was exchanged to 1 ml PBS, 1 % BSA was added for storage.
b) Coating of fluorophore particles with neutravidine: 1 mL of the fluorophore particle suspension (5 % w/v, with an outer layer of poly acrylic acid, PAA) was resuspensed in PBS buffer containing 10 mM N-hydroxy sucinimide (NHS) and 20 mM 1-Ethyl-3-[3-dimethylaminopropyl] carbodiimide hydrochloride (EDC), the mixture was incubated for 2 hours at room temperature. The not reacted EDC/NHS was removed by a centrifugation and wash cycle. 1 ml of a solution of 2 mg neutravidine (Pierce, USA) in carbonate buffer (pH 8.5) were used to resuspend the particles. The suspension was incubated for 3 hours at room temperature. The uncoated neutravidine was removed by a centrifugation and wash cycle and the particles were finally resuspended in 1 ml PBS with 1 % BSA.
c) Coating of fluorophore particles with biotinylated antibody: 1 mL of the fluorophore particle suspension (5 % w/v) were incubated with 100 µL of the biotinylated antibody anti-hCG-b antibody (1 mg/mL), for 2 hours at room temperature. The excess of antibody was removed by repeated centrifugation and wash cycles, the coated capsules were finally resuspended in PBS. The capsules are ready to use or storage after the addition of 1 % BSA.

### Example 5

### Use of capsules coated with affinity molecules in bioassays

### 1) Solid Phase Immunoassay for human chorionic gonadotropin (hCG)

a) Coating: 100 µl of a solution of 25 µg/mL anti-hCG antibody (Clone ME 107, Dunn Labortechnik, Asbach, Germany) in carbonate buffer (100 mM, pH 9.6) per well were incubated in a 96 well multi titer plate (Costar Maxi Sorb polystyrene plate) for 3 hours. The plate was washed with water and blocked with 300 µL of a 1 % BSA solution in PBS per well for 1 hour.
b) Sample incubation: 200 µl of hCG samples (Sigma) with dilutions from 0.1 µU to 100 µU per ml were incubated for 2 hours at room temperature. The plate was washed twice with water.
c) Label incubation: The fluorophore particle suspension (5 % w/v) with the biotinylated anti-hCG-b antibody conjugated via neutravidin was diluted 1/1000 with a PBS buffer containing 1 % BSA. 200 µL per well of the dilution were incubated for 2 hours at room temperature. The plate was washed twice with water and 300 µL of ethanol (96 %) were added and incubated for 2 minutes under shaking.
d) Fluorescent measurement: The content of released fluorophore from the label capsules was measured with a Fluorescent ELISA plate reader. The values of the samples (B) were set in relation to the values measured without sample (B₀).

### 2) Test Tube Assay for Fatty Acid- Binding Protein (FABP)

a) Coating: 400 µl of a solution of 1 mg/mL anti-FABP antibody in carbonate buffer (100 mM, pH 9.6). were incubated in a 5 mL Costar Maxi Sorb polystyrene tube for 3 hours (only the first cm of the bottom was in contact with the solution). The test tube was washed with water and blocked with 2 ml of a 1 % BSA solution in PBS for 1 hour (the complete inner wall of the tube was blocked).
b) Sample incubation: 200 µL of FABP samples with dilutions of 0.1 µg to 50 µg per ml were incubated in the test tube for 2 hours at room temperature. The tubes were washed twice with water.
c) Label incubation: The fluorophore particle suspension (5 % w/v) with the direct adsorbed monoclonal anti-FABP antibody was diluted 1/1000 with a PBS buffer containing 1 % BSA. 300 µl of the dilution were incubated in the test tube for 2 hours at room temperature. The test tube was washed twice with water and 500 µL of ethanol (96 %) was added and incubated for 2 minutes under shaking.
d) Fluorescent measurement: The content of released fluorophore from the label capsules was measured with a spectrofluorimeter. The values of the samples (B) were set in relation to the values measured without sample (B₀).

## Claims

1. Method for optical, electrochemical or chemical detection of one or more target molecules in a sample using capsules encapsulating solid particles of signal-generating organic substances and carrying on the outer surface affinity molecules for specific recognition of and binding to the target molecules and comprising the steps:
i) incubating the target molecules with capsules comprising a capsule wall which is selected from one or multiple polyelectrolyte layers, encapsulating water insoluble solid particles of signal-generating organic substances, and carrying on the outer capsule surface affinity molecules for specific recognition of and binding to target molecules,
ii) separating capsules with affinity-target molecule complexes on their surface from capsules with unreacted affinity molecules on their surface,
iii) releasing and dissolving the encapsulated solid particles of the signal-generating organic substance by treating the obtained capsules carrying affinity-target molecule complexes on their surface with physical or chemical means,
iv) detecting or quantifying the signal which is generated by the released and dissolved signal-generating substances and which is directly or indirectly related to the amount of the target molecules.

2. Method according to claim 1, **characterized in that** the affinity molecules are selected from the group of peptides or proteins, nucleic acids, carbohydrates, ligands with low molecular weight and molecular imprinted polymers (MIPs) or mixtures thereof.

3. Method according to claim 2, **characterized in that** the peptides or proteins are selected from the group of antibodies, receptors, antigens, lectins, avidins, oligopeptides, lipopoteins, glycoproteins, peptide hormones and allergenes or parts thereof.

4. Method according to claim 2, **characterized in that** the nucleic acids are selected from the group of single or double stranded DNA, RNAs, oligonucleotides, ribozymes, aptamers and parts thereof.

5. Method according to claim 2, **characterized in that** the carbohydrates are selected from the group of mono, oligo and polysaccharides, glycolipids, proteo-polysaccharides and parts thereof.

6. Method according to claim 2, **characterized in that** the low molecular weight ligands are biotin or biotin derivatives, steroids or hormones, a cofactor or coenzyme, activator, inhibitor, pseudosubstrate or prosthetic group of an enzyme, a drug, a pesticide, an allergen or digoxine or a hapten.

7. Method according to claim 2, **characterized in that** the affinity molecules are conjugated or bound directly or via linker molecules to the outer surface of the capsules.

8. Method according to claim 6, **characterized in that** the linker molecule is a biomolecule, preferably avidine, streptavidine, neutravidine, protein A, protein G, lectine or a low molecular crosslinker.

9. Method according to claim 1, **characterized in that** the encapsulated solid particle of signal-generating organic substance is a crystal, amorphous or a lyophilised particle, a spray dried particle, a milled particle or mixtures thereof.

10. Method according to claim 1, **characterized in that** the encapsulated solid particle has a particle size from 10 nm to 10 µm, preferably smaller than 1µm.

11. Method according to claim 1 **characterized in that** the encapsulated solid particle of signal-generating organic substance is a low molecular substance selected from the group of fluorophores, luminophores, chromophores, enzyme substrates, prosthetic groups, or redox active substances selected from redox mediators, electrodeactive substances.

12. Method according to claim 1, **characterized in that** the encapsulated solid particle of signal-generating organic substance is a high-molecular substances selected from the group of enzymes and their precursors, bioluminogenic and fluorogenic proteins, nucleic acids, ribozymes and aptamers. goups, wherein R and R¹ are independently from one another hydrogen or a linear or branched alkyl group.

13. Method according to claim 1, **characterized in that** the polyelectrolytes in one layer and/or between the layers are cross-linked.

14. Method according to claim 1 , **characterized in that** the polyelectrolytes are selected from organic polymers, biopolymers and mixtures thereof.

15. Method according to claim 14, **characterized in that** the organic polymer is a polymer selected from polyamin (PAH), polysulfonic acid (PSS), polyglycolic acid (PGA), polylactic acid (PLA), polyamides, poly-2-hydroxy butyrate (PHB), polycaprolactone (PCL), fluorescent labelled polymers and their copolymers and/or mixtures thereof.

16. Method according to claim 14, **characterized in that** the biopolymer is selected from polyaminoacids, especially peptides, polylysine, from polycarbohydrates, especially dextrin, pectin, alginate, glycogen, amylose, chitin, chondroitin, hyaluronic acid, from polynucleotides, especially DNA, RNA, oligonucleotides, and from modified biopolymers, especially carboxymethyl cellulose, carboxymethyl dextran, lignin sulfonates.

17. Method according to claim 1 **characterized in that** the release of the signal-generating substances from the capsules is achieved by chemical means, preferably by treating with an organic solvent or with an enzyme, ribozyme or aptamer or by changing the pH or ionic strength value.

18. Method according to claim 17, **characterized in that** the organic solvent is selected from the group of alcohols, especially ethanol and methanol, ketones, especially acetone, esters, especially ethylester, aromates, especially toluene, sulfoxides, especially DMSO and ethers, especially dimethylether, chloroform or from mixtures of organic solvents with one another or with water.

19. Method according to claim 1 **characterized in that** the release of the signal-generating substances from the capsules is achieved by physical means, preferably by ultrasonic disintegration, electric impulse or osmotic shock.

20. Kit for optical, electrochemical or chemical detection of target molecules in a sample comprising capsules comprising a capsule wall which is selected from one or multiple polyelectrolyte layers, encapsulating water insoluble solid particles of signal-generating organic substances, and carrying on its outer surface affinity molecules for specific recognition of and binding to target molecules.

21. Kit according to claim 20 comprising a dipstick.

22. Kit for optical, electrochemical or chemical detection of target molecules in a sample comprising
a) capsules comprising a capsule wall which are selected from one or multiple polyelectrolyte layers encapsulating water insoluble solid particles of signal-generating organic substances, being designated to bind affinity molecules
b) agents for the modification of affinity molecules to make them suitable to bind to the surface of the capsules
c) agents for performing the binding reaction between the capsules and the affinity molecules.

23. Kit according to claim 22 comprising a dipstick.

## Patentansprüche

1. Verfahren zum optischen, elektrochemischen oder chemischen Nachweis eines oder mehrerer Zielmoleküle in einer Probe unter Verwendung von Kapseln, welche feste Teilchen von signalerzeugenden organischen Substanzen einkapseln und an der äußeren Oberfläche Affinitätsmoleküle zur spezifischen Erkennung von und Bindung an die Zielmoleküle tragen, umfassend die Schritte:
i) Inkubieren der Zielmoleküle mit Kapseln, welche eine Kapselwandung umfassen, die ausgewählt aus einer oder mehreren Polyelektrolyt-Schichten ist und welche wasserunlösliche feste Teilchen signalerzeugender Substanzen einkapseln und die an ihrer äußeren Oberfläche Affinitätsmoleküle zur spezifischen Erkennung von und Bindung an Zielmoleküle tragen,
ii) Abtrennen des resultierenden Komplexes aus Ziel- und Affinitätsmolekül von den Kapseln mit unumgesetzten Affinitätsmolekülen auf deren Oberfläche,
iii) Freisetzen und Lösen der eingekapselten festen Teilchen der signalerzeugenden Substanzen durch Behandeln des erhaltenen Ziel/Affinitäts-Komplexes mit physikalischen Mitteln ausgewählt aus Ultrashall-Zermahlung oder elektrischem Impuls oder chemischen Mitteln ausgewählt aus Behandeln mit einem organischen Lösungsmittel oder mit einem Enzym, Ribozym oder Aptamer oder durch Ändern des pH-Werts
iv) Nachweisen oder Quantifizieren des Signals, das durch die freigesetzten und gelösten signalerzeugenden Substanzen erzeugt wird und das in direkter oder indirekter Beziehung zur Menge der Zielmoleküle steht.

2. Verfahren nach Anspruch 1, **dadurch gekennzeichnet, dass** die Affinitätsmoleküle ausgewählt sind aus der Gruppe der Peptide oder Proteine, Nucleinsäuren, Kohlenhydrate, Liganden mit niedrigem Molekulargewicht und molekular aufgedruckten Polymeren (MIPs) oder Mischungen derselben.

3. Verfahren nach Anspruch 2, **dadurch gekennzeichnet, dass** die Peptide oder Proteine ausgewählt sind aus der Gruppe der Antikörper, Rezeptoren, Antigene, Lektine, Avidine, Oligopeptide, Lipoproteine, Glycoproteine, Peptidhormone und Allergene oder Teilen derselben.

4. Verfahren nach Anspruch 2, **dadurch gekennzeichnet, dass** die Nucleinsäuren ausgewählt sind aus der Gruppe der einzel- oder doppelsträngigen DNAs, RNAs, Oligonucleotide, Ribozyme, Aptamere und Teilen derselben.

5. Verfahren nach Anspruch 2, **dadurch gekennzeichnet, dass** die Kohlenhydrate ausgewählt sind aus der Gruppe der Mono-, Oligo- und Polysaccharide, Glycolipide, Proteopolysaccharide und Teilen derselben.

6. Verfahren nach Anspruch 2, **dadurch gekennzeichnet, dass** der Ligand mit niedrigem Molekulargewicht Biotin oder ein Biotin-Derivat, ein Steroid oder Hormon, ein Cofaktor oder Coenzym, ein Aktivator, Inhibitor, Pseudosubstrat oder eine prosthetische Gruppe eines Enzyms, ein Arzneimittel, ein Pestizid, ein Allergen oder Digoxin oder ein Hapten ist.

7. Verfahren nach Anspruch 2, **dadurch gekennzeichnet, dass** die Affinitätsmoleküle an die äußere Oberfläche der Kapseln konjugiert oder direkt oder über Linker-Moleküle daran gebunden sind.

8. Verfahren nach Anspruch 6, **dadurch gekennzeichnet, dass** das Linker-Molekül ein Biomolekül ist, vorzugsweise Avidin, Streptavidin, Neutravidin, Protein A, Protein G, Lektin oder ein niedermolekularer Vernetzer.

9. Verfahren nach Anspruch 1, **dadurch gekennzeichnet, dass** die eingekapselten festen Teilchen signalerzeugender Substanzen Kristalle, amorphe oder lyophilisierte Teilchen, sprühgetrocknete Teilchen, gemahlene Teilchen oder Mischungen derselben sind.

10. Verfahren nach Anspruch 1, **dadurch gekennzeichnet, dass** die eingekapselten festen Teilchen eine Teilchengröße von 10 nm bis 10 µm aufweisen, vorzugsweise kleiner als 1 µm.

11. Verfahren nach Anspruch 1, **dadurch gekennzeichnet, dass** die eingekapselten festen Teilchen signalerzeugender Substanzen niedermolekulare Substanzen sind, ausgewählt aus der Gruppe der Fluorophore, Luminophore, Chromophore, Enzymsubstrate, prosthetischen Gruppen, oder redoxaktive Substanzen, ausgewählt aus Redoxvermittlern, elektrodenaktiven Substanzen.

12. Verfahren nach Anspruch 1, **dadurch gekennzeichnet, dass** die eingekapselten festen Teilchen signalerzeugender Substanzen hochmolekulare Substanzen sind, ausgewählt aus der Gruppe der Enzyme und ihrer Vorläufer, bioluminogenen und fluorogenen Proteine, Nucleinsäuren, Ribozyme und Aptamere.

13. Verfahren nach Anspruch 1, **dadurch gekennzeichnet, dass** die Polyelektrolyten in einer Schicht und/oder vernetzt zwischen den Schichten voliegen.

14. Verfahren nach Anspruch 1, **dadurch gekennzeichnet, dass** die Polyelektrolyte aus organischen Polymeren, Biopolymeren und Mischungen derselben ausgewählt sind.

15. Verfahren nach Anspruch 14, **dadurch gekennzeichnet, dass** das organische Polymer ein aus Polyamin (PAH), Polysulfonsäure (PSS), Polyglycolsäure (PGA), Polymilchsäure (PLA), Polyamiden, Poly-2-hydroxybutyrat (PHB), Polycaprolacton (PCL), fluoreszenzmarkierten Polymeren und ihren Copolymeren und/oder Mischungen derselben ausgewähltes Polymer ist.

16. Verfahren nach Anspruch 14, **dadurch gekennzeichnet, dass** das Biopolymer ausgewählt ist aus Polyaminosäuren, insbesondere Peptiden, Polylysin, aus Polykohlenhydraten, insbesondere Dextrin, Pektin, Alginat, Glycogen, Amylose, Chitin, Chondroitin, Hyaluronsäure, aus Polynucleotiden, insbesondere DNA, RNA, Oligonucleotiden, sowie aus modifizierten Biopolymeren, insbesondere Carboxymethylcellulose, Carboxymethyldextran oder Ligninsulfonate.

17. Verfahren nach Anspruch 1, **dadurch gekennzeichnet, dass** die Freisetzung der signalerzeugenden Substanzen aus den Kapseln durch chemische Methoden erreicht wird, vorzugsweise durch Behandeln mit einem Enzym,Ribozym oder Aptamer oder durch Änderung des pH-wertes oder ionische ...

18. Verfahren nach Anspruch 17, **dadurch gekennzeichnet, dass** das organische Lösungsmittel ausgewählt ist aus der Gruppe der Alkohole, insbesondere Ethanol und Methanol, Ketone, insbesondere Aceton, Ester, insbesondere Ethylester, Aromaten, insbesondere Toluol, Sulfoxide, insbesondere DMSO, sowie Ether, insbesondere Dimethylether, Chloroform oder aus Mischungen organischer Lösungsmittel untereinander oder mit Wasser.

19. Verfahren nach Anspruch 1, **dadurch gekennzeichnet, dass** die Freisetzung der signalerzeugenden Substanzen aus den Kapseln durch physikalische Methoden erreicht wird, vorzugsweise durch ultrasonische Disintegration, elektrische Impulse oder osmotischen Schock.

20. Kit für den optischen, elektrochemischen oder chemischen Nachweis von Zielmolekülen in einer Probe, umfassend Kapseln umfassend eine Kapselwandung, welche ausgewählt sind aus einer oder mehreren Polyelektroly-Schichten, die wasserunlösliche feste Teilchen signalerzeugender Substanzen einkapseln, die freigesetzt und gelöst werden können, und an ihrer äußeren Oberfläche Affinitätsmoleküle zum spezifischen Erkennen und Binden an ein Zielmolekül tragen.

21. Kit nach Anspruch 20, umfassend einen Eintauchstreifen.

22. Kit für den optischen, elektrochemischen oder chemischen Nachweis von Zielmolekülen in einer Probe, umfassend
a) Kapseln, umfassend eine Kapselwandung, welche ausgewählt sind aus einer oder mehreren Polyelektroly-Schichten, die wasserunlösliche, feste Teilchen signalerzeugender Substanzen einkapseln, die freigesetzt und gelöst werden können, und Affinitätsmoleküle binden sollen;
b) Agenzien zur Modifizierung von Affinitätsmolekülen, so daß sie zur Bindung an die Oberfläche der Kapseln geeignet sind;
c) Agenzien zur Durchführung der Bindungsreaktion zwischen den Kapseln und Affinitätsmolekülen.

23. Kit nach Anspruch 22, umfassend einen Eintauchstreifen.

## Revendications

1. Procédé de détection optique, électrochimique ou chimique d'une ou plusieurs molécules cibles dans un échantillon réalisé en utilisant des capsules encapsulant des particules solides de substances organiques générant un signal, et portant sur leur surface extérieure des molécules d'affinité destinées à une reconnaissance spécifique des molécules cibles et se liant à celles-ci, comprenant les étapes suivantes :
i) incuber les molécules cibles avec des capsules comprenant une paroi de capsule qui est choisie parmi une ou plusieurs couches de polyélectrolyte, encapsulant des particules solides insolubles dans l'eau de substances organiques générant un signal et portant sur leur surface extérieure de capsule des molécules d'affinité pour la reconnaissance spécifique des molécules cibles et se liant à celles-ci,
ii) séparer les capsules contenant des complexes molécule d'affinité-molécule cible sur leur surface vis-à-vis des capsules comportant des molécules d'affinité n'ayant pas réagi sur leur surface,
iii) libérer et dissoudre les particules solides encapsulées de substance organique générant un signal en traitant les capsules obtenues comportant des complexes molécule d'affinité-molécule cible sur leur surface par des moyens physiques ou chimiques,
iv) détecter ou quantifier le signal qui est produit par les substances générant un signal libérées et dissoutes et qui est relié directement ou indirectement à la quantité de molécules cibles.

2. Procédé selon la revendication 1, **caractérisé en ce que** les molécules d'affinité sont choisies parmi le groupe constitué des peptides ou des protéines, des acides nucléiques, des carbohydrates, des ligands à bas poids moléculaire et des polymères à empreinte moléculaire (MIP) ou des mélanges d'entre eux.

3. Procédé selon la revendication 2, **caractérisé en ce que** les peptides ou les protéines sont choisis parmi le groupe comprenant les anticorps, les récepteurs, les antigènes, les lectines, les avidines, les oligopeptides, les lipoprotéines, les glycoprotéines, les hormones peptidiques et les allergènes ou des parties de ceux-ci.

4. Procédé selon la revendication 2, **caractérisé en ce que** les acides nucléiques sont choisis parmi le groupe constitué d'ADN à simple ou à double brin, d'ARN, d'oligonucléotides, de ribozymes, d'aptamères et de parties de ceux-ci.

5. Procédé selon la revendication 2, **caractérisé en ce que** les carbohydrates sont choisis parmi le groupe constitué des mono-, oligo- et polysaccharides, des glycolipides, des protéo-polysaccharides et des parties de ceux-ci.

6. Procédé selon la revendication 2, **caractérisé en ce que** les ligands à bas poids moléculaire sont de la biotine ou des dérivés de la biotine, des stéroïdes ou des hormones, un cofacteur ou un coenzyme, un activateur, un inhibiteur, un pseudo-substrat ou un groupe prosthétique d'un enzyme, un principe actif, un pesticide, un allergène ou digoxine ou un haptène.

7. Procédé selon la revendication 2, **caractérisé en ce que** les molécules d'affinité sont conjuguées ou liées directement ou via des molécules de linker à la surface extérieure des capsules.

8. Procédé selon la revendication 6, **caractérisé en ce que** la molécule de linker est une biomolécule, de préférence de l'avidine, de la streptavidine, de la neutravidine, de la protéine A, de la protéine G, de la lectine ou un agent réticulant à bas poids moléculaire.

9. Procédé selon la revendication 1, **caractérisé en ce que** la particule solide encapsulée de substance organique générant un signal est un cristal, une particule amorphe ou lyophilisée, une particule pulvérisée séchée, une particule broyée ou des mélanges d'entre eux.

10. Procédé selon la revendication 1, **caractérisé en ce que** la particule solide encapsulée a une taille de particule de 10 nm à 10 µm, de préférence inférieure à 1 µm.

11. Procédé selon la revendication 1, **caractérisé en ce que** la particule solide encapsulée de substance organique générant un signal est une substance à bas poids moléculaire choisie parmi le groupe constitué des fluorophores, des luminophores, des chromophores, des substrats d'enzyme, des groupes prosthétiques, ou des substances ayant une activité redox choisies parmi les médiateurs redox, les substances actives à l'électrode.

12. Procédé selon la revendication 1, **caractérisé en ce que** la particule solide encapsulée de substance organique générant un signal est une substance à haut poids moléculaire choisie parmi le groupe constitué des enzymes et de leurs précurseurs, les protéines bioluminogènes et fluorogènes, les acides nucléiques, les rybozymes et les aptamères.

13. Procédé selon la revendication 1, **caractérisé en ce que** les polyélectrolytes dans une couche et/ou entre les couches sont réticulées.

14. Procédé selon la revendication 1, **caractérisé en ce que** les poly-électrolytes sont choisis parmi les polymères organiques, les bio-polymères et les mélanges d'entre eux.

15. Procédé selon la revendication 14, **caractérisé en ce que** le polymère organique est choisi parmi le groupe comprenant le polyamine (PAH), l'acide polysulfonique (PSS), l'acide polyglycolique (PGA), l'acide polylactique (PLA), les polyamides, le poly-2-hydroxybutyrate (PHB), le polycaprolactone (PCL), les polymères marqués en fluorescence et leurs copolymères et/ou des mélanges d'entre eux.

16. Procédé selon la revendication 14, **caractérisé en ce que** le biopolymère est choisi parmi le groupe comprenant les polyaminoacides, en particulier les peptides, les polycarbohydrates, notamment la dextrine, la pectine, l'alginate, le glycogène, l'amylose, la chitine, la chondroïtine, l'acide hyaluronique, les polynucléotides, notamment l'ADN, l'ARN, les oligonucléotides et les biopolymères modifiés, notamment la carboxyméthyl cellulose, le carboxyméthyl dextrane, les sulfonates de lignine.

17. Procédé selon la revendication 1, **caractérisé en ce que** la libération des substances générant un signal à partir des capsules s'effectue par des moyens chimiques, de préférence par traitement avec un solvant organique ou avec un enzyme ou un aptamère ou en modifiant le pH ou la force ionique.

18. Procédé selon la revendication 17, **caractérisé en ce que** le solvant organique est choisi dans le groupe constitué des alcools, notamment l'éthanol et le méthanol, des cétones, notamment l'acétone, des esters, notamment l'éthylester, des aromatiques, notamment le toluène, des sulfoxydes, notamment le DMSO et des éthers, notamment le diméthyléther, le chloroforme ou dans les mélanges de solvants organiques entre eux ou avec de l'eau.

19. Procédé selon la revendication 1, **caractérisé en ce que** la libération des substances générant un signal à partir des capsules s'effectue par des moyens physiques, de préférence par une désintégration par ultrasons, une impulsion électrique ou un choc osmotique.

20. Kit de détection optique, électrochimique ou chimique des molécules cibles dans un échantillon, comprenant des capsules comportant une paroi de capsule qui est choisie parmi une ou plusieurs couches de polyélectrolyte, encapsulant des particules solides insolubles dans l'eau de substances organiques générant un signal, et comportant sur sa surface extérieure des molécules d'affinité pour une reconnaissance spécifique des molécules cibles et se liant à celles-ci.

21. Kit selon la revendication 20, comprenant une réglette graduée.

22. Kit de détection optique, électrochimique ou chimique de molécules cibles dans un échantillon comprenant :
a) des capsules comprenant une paroi de capsule qui est choisie parmi une ou plusieurs couches de polyélectrolyte, encapsulant des particules solides insolubles dans l'eau de substances organiques générant un signal, destinées à se lier à des molécules d'affinité,
b) des agents de modification des molécules d'affinité pour les rendre aptes à se lier à la surface des capsules,
c) des agents pour réaliser la réaction de liaison entre les capsules et les molécules d'affinité.

23. Kit selon la revendication 22, comprenant une réglette graduée.
